# FASCICULE DE BREVET EUROPEEN

(11) **EP 1 121 451 B1**
(45) Date de publication et mention de la délivrance du brevet: **01.02.2006**
(21) Numéro de dépôt: 99970424.0
(22) Date de dépôt: 08.10.1999
(51) Int. Cl.: C12N 15/82, A01H 5/00

(54) **PROCEDE D'OBTENTION DE PLANTES TRANSGENIQUES EXPRIMANT UNE PROTEINE A ACTIVITE PRODUCTRICE DE PEROXYDE D'HYDROGENE PAR TRANSFORMATION PAR AGROBACTERIUM RHIZOGENES**
VERFAHREN ZUR HERSTELLUNG VON TRANSGENEN PFLANZEN, DIE EIN PROTEIN EXPRIMIEREN, DAS EINE WASSERSTOFF-PEROXID PRODZIERENDE AKTIVITÄT BESITZT, MITTELS TRANSFORMATION DURCH AGROBAKTERIUM RHIZOGENES
METHOD FOR OBTAINING TRANSGENIC PLANTS EXPRESSING A PROTEIN WITH ACTIVITY PRODUCING HYDROGEN PEROXIDE BY TRANSFORMATION BY AGROBACTERIUM RHIZOGENES

(30) Priorité: 09.10.1998 FR 9812704
(43) Date de publication de la demande: 08.08.2001
(73) Titulaire: Biogemma, 75001 Paris (FR)
(72) Inventeur: PAGNIEZ, Michel, F-31820 Pibrac (FR); GRISON, René, F-31750 Escalquens (FR); TOPPAN, Alain, F-31700 Cornebarrieu (FR)
(74) Mandataire: Flesselles, Bruno F.G.
(86) Numéro de dépôt international: PCT/FR1999/002412
(87) Numéro de publication internationale: WO 2000/022148

(56) Documents cités:
- EP-A- 0 262 972
- EP-A- 0 687 730
- EP-A- 0 716 147
- WO-A-92/01792
- WO-A-94/13790
- WO-A-97/37012
- WO-A-98/51806
- HATAMOTO, H., ET AL.: "Recovery of morphologically normal transgenic tobacco from hairy roots co-transformed with Agrobacterium rhizogenes and a binary vector plasmid" PLANT CELL REPORTS, vol. 9, 1990, pages 88-92, XP002107166
- BOULTER M E ET AL: "TRANSFORMATION OF BRASSICA NAPUL L.(OILSEED RAPE) USING AGROBACTERIUM TUMEFACIENS AND AGROBACTERIUM RHIZOGENES - A COMPARISON" PLANT SCIENCE, vol. 70, 1990, pages 91-99, XP000775906 ISSN: 0168-9452
- CHEMICAL ABSTRACTS, vol. 121, no. 23, 5 décembre 1994 (1994-12-05) Columbus, Ohio, US; abstract no. 276934, WEBB, K. J, ET AL.: "Expression of GUS in primary transformants and segregation patterns of GUS, TL- and TR-DNA in the T1 generation of hairy root transformants of Lotus corniculatus" XP002107276 & TRANSGENIC RES. (1994), 3(4), 232-40,
- SHAHIN, E.A., ET AL.: "Transformation of cultivated tomato by a binary vector in Agrobacterium rhizogenes: transgenic plants with normal phenotypes harbor binary vector T-DNA, but no Ri-plasmid T-DNA" THEOR. APPL. GENET., vol. 72, no. 6, 1986, pages 720-777, XP002107167 cité dans la demande
- SIMPSON, R.B., ET AL.: "A disarmed binary vector from Agrobacterium tumefaciens functions in Agrobacterium rhizogenes" PLANT MOLECULAR BIOLOGY, vol. 6, 1986, pages 403-415, XP002107168
- ZHANG Z ET AL: "Germin-like oxalate oxidase, a H2O2-producing enzyme, accumulates in barley attacked by the powdery mildew fungus" PLANT JOURNAL, vol. 8, no. 1, 1995, pages 139-145, XP002084833 ISSN: 0960-7412
- DATABASE SCISEARCH [Online] AN 96:394736, GRISON, R., ET AL.: "FIELD TOLERANCE TO FUNGAL PATHOGENS OF BRASSICA-NAPUS CONSTITUTIVELY EXPRESSING A CHIMERIC CHITINASE GENE" XP002107279 & NATURE BIOTECHNOLOGY, (MAY 1996) VOL. 14, NO. 5, PP. 643-646.,

## Description

L'invention concerne le domaine de la transformation génétique des plantes par *Agrobacterium rhizogenes*, combinée à l'utilisation d'un gène codant pour une protéine à activité productrice de peroxyde d'hydrogène et notamment l'oxalate oxydase.

L'objet de la présente invention est un procédé d'obtention de plantes transgéniques, caractérisé en ce qu'il utilise une transformation par *Agrobacterium rhizogenes* associée à un tri visuel des événements de transformation, basé sur la coloration, simple à mettre en oeuvre et rapide, des racines transformées exprimant le transgène.

Depuis que les premiers essais aux champs, donc hors confinement, de plantes transgéniques ont eu lieu en 1986, l'usage d'un gène de résistance à un antibiotique comme gène de sélection a fait l'objet de nombreuses critiques (Casse-Delbart et Tepfer, Biofutur, (1990), Juin ; 56-59, ainsi que Bryant et Leather, Tibtech, (1992), 10, 274-275). Bien que les possibilités d'une transmission de gènes de la plante transgénique à des bactéries du sol n'aient pas été mises en évidence, l'utilisation de gènes de résistance à des antibiotiques est très mal perçue par certains auteurs (Heinemann, TIG, (1991), 7, 181-185).

De nombreux substituts au gène de résistance à la kanamycine ont été proposés (Ratner, Bio-Technology, (1989), 7, 337-341) mais la plupart de ces gènes de résistance à un autre antibiotique ou à un herbicide, conduisent à des objections semblables ou à des difficultés techniques de mise en oeuvre.

L'utilisation de l'oxalate oxydase comme gène de sélection offre une alternative aux systèmes précédents. Mise en évidence chez certaines plantes, cette protéine est capable de dégrader l'acide oxalique, qui est une phytotoxine produite lors de l'infection par de nombreux pathogènes de plantes.

Une première application de l'oxalate oxydase a été la production de plantes transgéniques résistantes aux pathogènes du genre *Sclerotinia* (WO 92/14824).

La demande de brevet WO 94/13790 décrit une nouvelle utilisation de l'oxalate oxydase en remplacement de la kanamycine, dans un système de pression sélective des transformants. Selon le procédé décrit dans WO 94/13790, seuls les transformants (cals) contenant le gène de l'oxalate oxydase, survivent à une dose subléthale déterminée d'acide oxalique, présente dans le milieu. Mais la préparation des milieux de sélection (dose d'acide oxalique, chélateurs de calcium) est lourde et contraignante.

Jusqu'ici, l'oxalate oxydase était utilisée couplée à l'agent toxique, l'acide oxalique, dans une perspective de pression sélective, alors que dans le procédé de la présente demande il est utilisé comme simple marqueur pour un tri visuel des transformants. Pourtant, cette protéine ne semblait pas être *a priori* un bon candidat comme marqueur, puisqu'elle était fortement exprimée chez des plantes infectées par un pathogène, d'où un risque important de fausser les résultats (faux positifs, par exemple). De plus, l'enseignement de la demande de brevet WO 94/13790 n'encourageait pas l'homme du métier à utiliser un test colorimétrique pour la sélection des transformants, puisqu'un tel test permet de sélectionner des événements individualisés (cellules transformées) et non des événements éparpillés dans un matériel hétérogène comme le cal, issu de la transformation par *Agrobacterium tumefaciens* et contenant en mélange des cellules transformées et des cellules non-transformées.

La présente invention propose donc un procédé d'obtention de plantes transgéniques, avantageux en ce qu'il supprime les contraintes techniques du procédé sus-décrit par la combinaison des caractéristiques *d'Agrobacterium rhizogenes* et d'une protéine à activité productrice de H₂O₂. En particulier, les inventeurs exploitent la formation de racines induite par *Agrobacterium rhizogenes* et le phénotype des transformants (feuilles gaufrées, racines plagiotropes, entre-noeuds raccourcis) en association avec la production de H₂O₂ (peroxyde d'hydrogène) par la protéine pour la mise au point d'un nouveau système de sélection de transformants homogènes. Dans ce système le peroxyde d'hydrogène produit est révélé en présence de peroxydase, pour produire une coloration des échantillons de racines transformées.

Selon un premier aspect, la présente invention a pour objet un procédé d'obtention de plantes transgéniques qui comprend les étapes suivantes de :
(a) transformation de cellules végétales par *Agrobacterium rhizogenes* contenant un vecteur porteur d'un gène codant pour une protéine à activité productrice de H₂O₂ ;
(b) sélection des transformants qui contiennent et expriment ce gène par un test colorimétrique à la peroxydase ;
(c) régénération des plantes à partir des racines sélectionnées et contrôle de l'expression des plantules obtenues par un test colorimétrique à la peroxydase ;
(d) tri selon leur phénotype et éventuellement validation par analyse moléculaire de la descendance des plantes transgéniques obtenues, permettant la sélection ou la confirmation de plantes contenant seulement le transgène et non l'ADN-T propre à *Agrobacterium rhizogenes.*

Le test colorimétrique à la peroxydase mis en oeuvre dans les étapes b) et c) du procédé de l'invention consiste à incuber un échantillon de tissus végétaux prélevés en présence d'un milieu contenant un substrat de la protéine à activité productrice de H₂O₂, tel que par exemple l'acide oxalique et à relever la formation de H₂O₂ à l'aide de la peroxydase en présence d'un substrat approprié dont l'oxydation s'accompagne d'un changement de coloration. La concentration en substrat dans le milieu d'incubation n'est pas critique puisque le système de tri visuel sur échantillon selon la présente invention ne requiert pas la survie des cellules, alors que c'est le cas pour le tri sélectif "vital" des cellules transformées décrit dans la demande WO 94/13790. Dans le cas présent, la concentration en substrat est avantageusement une concentration saturante pour que l'activité productrice de H₂O₂ soit favorisée et les produits facilement révélés à la peroxydase, sous forme d'une coloration bleue soutenue. L'enseignement de la demande WO 94/13790 n'indique pas l'utilisation de concentration en acide oxalique supérieure à la dose subléthale exemplifiée, qui est de 3mM ; or il s'avère que les concentrations optimales de substrat pour le tri visuel sont bien supérieures à cette concentration critique. A titre d'exemple, les concentrations d'acide oxalique peuvent être comprises dans une large gamme allant de 5 à 50mM et préférentiellement de 10 à 20 mM, en particulier 15 mM.

Le test colorimétrique effectué à l'étape b) du procédé est avantageusement réalisé sur un échantillon de la racine. Il peut également être effectué sur le milieu liquide d'incubation avantageusement après décontamination des explants (élimination des agrobactéries), sachant que la protéine, telle que l'oxalate oxydase, peut diffuser à partir du lieu où elle est exprimée. De même, une révélation sur empreinte laissée par les explants transformés sur un milieu gélosé ou sur une membrane, est également possible.

Le test colorimétrique effectué à l'étape c) du procédé peut être effectué sur un échantillon de tissu végétal des plantes régénérées.

Selon l'invention, les cellules végétales sont transformées par *Agrobacterium rhizogenes* contenant un ADN recombinant comprenant un gène codant pour une protéine à activité productrice de H₂O₂ dans un contexte qui permet son expression dans le végétal. *Agrobacterium rhizogenes,* qui est décrite par exemple par Tepfer, Physiologica Plantarium, (1990), vol. 79 p. 140-146, est une bactérie susceptible d'infecter lesdites cellules végétales en permettant l'intégration, dans le génome de ces dernières, des séquences d'ADN d'intérêt initialement contenues dans l'ADN-T *d'Agrobacterium rhizogenes.* En fait, la transformation des cellules végétales utilise un système binaire (Watson in Genetic engineering of crop plants, Butterworths, 1990) comprenant deux vecteurs : le premier étant le pRi propre à *Agrobacterium rhizogenes* et responsable de la formation des racines transformées ; le second vecteur, de type binaire (Bevan, Nuc. Acid. Res. (1984), 12 : 8711), contient le gène codant pour la protéine à activité productrice de H₂O₂ et préférentiellement pour l'oxalate oxydase ; ce gène sera dénommé ci-après gène de sélection.

La protéine codée par le gène de sélection est une protéine à activité productrice de H₂O₂. A titre d'exemples de telles protéines, on peut citer les NADPH oxydases, oxalate oxydases, amines oxydases, glucose oxydases, etc. (Bolwell et Wojtazek, Plant Mol. Plant Pathol.,(1998), 51, 347). Les gènes codant pour ces protéines peuvent être utilisés aux fins de l'invention.

De préférence, on utilisera un gène codant pour une protéine à activité oxydase, telle que par exemple l'oxalate oxydase d'orge (commercialisée par Boehringer, réf. 567698), de sorgho (Pundier, Phytochemistry, (1991), 30, 4, 1065) ou de mousse *Mnium menziesii* (Laker et al, Clinical Chemistry, (1980), 26, 7, 827).

Une protéine à activité oxalate oxydase particulièrement appréciée est la germine de blé, dont la séquence a été décrite par Dratewka-Kos, J. Biol. Chem, (1989), 264, 4896) et Lane, J. Biol. Chem.(1991), 266, 10461). Compte tenu de la dégénérescence du code génétique, il existe un grand nombre de séquences nucléotidiques codant pour l'oxalate oxydase, qui peuvent être également utilisées aux fins de l'invention.

L'ADN recombinant comprend le gène codant pour la protéine à activité productrice de H₂O₂, flanqué des moyens nécessaires à son expression, notamment un promoteur, un terminateur de transcription, et éventuellement une séquence codant pour un peptide d'adressage d'origine végétale.

Le promoteur est de préférence un promoteur constitutif fort, par exemple le promoteur 35S du virus de la mosaïque du chou-fleur.

De façon alternative, on peut utiliser le promoteur du gène codant pour le facteur d'élongation de plante, tel que le promoteur EF-1α décrit dans la demande de brevet W0 90/02172 ou par Axelos et al. Plant Mol. Biol., (1989), 219 : 1-2, 106. On peut également utiliser un promoteur spécifique d'un tissu, un promoteur actif au cours d'un stade précis du développement de la plante, ou un promoteur inductible dans des situations de stress, par exemple à la suite d'un choc thermique, d'une blessure ou de l'interaction entre la plante et des parasites (Kuhlemeier et al., Ann. Rev. Plant Physiol., (1987), 38 : 221), si la phase de sélection nécessite l'expression du gène producteur de H₂O₂ dans ces situations.

Parmi les autres promoteurs de transcription pouvant être utilisés, on peut citer notamment :
- le promoteur constitutif double 35S (pd35S) du CaMV, décrit dans l'article de Kay et al., Science, (1987), 236 : 4805 ;
- le promoteur chimérique super-promoteur PSP (Ni M et al., Plant J.,(1995), 7: 661) constitué de la fusion d'une triple répétition d'un élément activateur transcriptionnel du promoteur du gène de l'octopine synthase *d*'*Agrobacterium tumefaciens*, d'un élément activateur transcriptionnel du promoteur du gène de mannopine synthase et du promoteur mannopine synthase *d*'*Agrobacterium tumefaciens*;
- le promoteur actine du riz suivi de l'intron actine de riz (PAR-IAR) contenu dans le plasmide pAct1-F4 décrit par Mc Elroy et al., (Mol. Gen. Genet., (1991), 231 : 150);

On peut également utiliser des promoteurs spécifiques de la graine, notamment :
- le promoteur HMGW (High Molecular Weight Glutenine) d'orge décrit par Anderson et al., T.A.G., (1989), 77, 689-700 ;
- le promoteur du gène de γ-zéine de maïs (Pγ-zéine) contenu dans le plasmide pγ63, et permettant l'expression dans l'albumen des semences de maïs, décrit par Reina et al., Nucleic Acid Research, (1990), 18, 6426 ;
- le promoteur PCRU du gène de la cruciférine de radis permettant l'expression des séquences associées uniquement dans les semences (ou graines) de la plante transgénique obtenue ; ce promoteur est décrit par Depigny-This et al., Plant. Mol., Biol., (1992), 20,467-479 ;
- les promoteurs PGEA1 et PGEA6 correspondant à la région 5' non codante des gènes de la protéine de réserve de graines, GEA1 et GEA6, respectivement, d*'Arabidopsis thaliana* (Gaubier et al., Mol. Gen. Genet (1993), 238, p.409-418, et permettant une expression spécifique dans les graines ;

On utilise une séquence terminatrice, comportant des sites de polyadénylation, pouvant être isolée de gènes végétaux ou de gènes s'exprimant dans les végétaux, comme par exemple le terminateur du gène de la nopaline synthase *d'Agrobacterium tumefaciens* (Depicker et al., J. Mol. Appl. Genet (1982), 1, 561-573).

Parmi les séquences codant pour un peptide d'adressage d'origine végétale, pouvant être utilisées dans le cadre de l'invention, on peut citer :
- la séquence nucléotidique de 69 nucléotides codant pour le prépeptide (peptide signal) de 23 acides aminés de la sporamine A chez la patate douce, permettant l'entrée des polypeptides recombinants dans le système de sécrétion des cellules végétales transformées ;
- la séquence nucléotidique de 42 nucléotides codant pour le propeptide N-terminal d'adressage vacuolaire de 14 acides aminés de la sporamine A chez la patate douce, permettant l'accumulation des polypeptides recombinants dans les vacuoles des cellules végétales transformées ;
- la séquence de 111 nucléotides codant pour le prépropeptide de 37 acides aminés de la sporamine A constitué depuis l'extrémité N-terminale vers l'extrémité C-terminale des 23 acides aminés du peptide signal susmentionné suivis par les 14 acides aminés du propeptide susmentionné, ce prépropeptide permettant l'entrée de polypeptides recombinants dans le système de sécrétion et leur accumulation dans les vacuoles des cellules végétales transformées selon l'invention,
   les trois séquences susmentionnées étant décrites par Murakami et al., Plant Mol. Biol.,(1986), 7, 343-355 et par Matsuoka et al. dans Proc. Natl. Acad. Sci. USA, (1991), 88, 834-838.
- le propeptide carboxyterminal de la lectine d'orge décrit notamment par Schroeder et al. dans Plant Physiol., (1993), 101, 451-458 et par Bednarek et al., dans The Plant Cell, (1991), 3, 1195-1206;
- et le PRS (Pathogenesis Related Protein) de Cornelissen et al. Nature, (1986), 321, 531-532 permettant la sécrétion.

On peut également citer, parmi les séquences codant pour un peptide d'adressage, celles codant pour les peptides Lys-Asp-Glu-Leu (KDEL) ; Ser-Glu-Lys-Asp-Glu-Leu (SEKDEL) ; et His-Asp-Glu-Leu (HDEL) et permettant un adressage dans le réticulum endoplasmique.

Une bactérie, par exemple de l'espèce *Escherichia coli*, qui contient l'ADN recombinant défini ci-dessus avec les moyens permettant sa réplication peut servir au clonage de cet ADN recombinant. Une bactérie susceptible d'infecter une plante avec transfert de matériel génétique, *Agrobacterium rhizogenes,* qui contient cet ADN dans un contexte permettant sa réplication, servira à transformer des cellules végétales.

L'invention permet aussi d'obtenir une cellule végétale, caractérisée en ce qu'elle est transformée par l'ADN recombinant défini précédemment. Cette cellule végétale peut provenir d'une espèce de grande culture, telle que par exemple le maïs, le soja, le blé, l'orge, le colza, le tournesol et le pois ou d'une espèce potagère, telle que la laitue, le melon, la tomate, les choux, l'oignon et le maïs, maïs doux de préférence. Des espèces particulièrement appréciées sont le colza *Brassica napus*, le tournesol *Helianthus annuus*, le tabac *Nicotiana tabacum*, le choux fleur *Brassica oleracea* et la tomate *Lycopersicon esculentum*. De préférence, l'invention concerne des espèces qui n'expriment pas l'oxalate oxydase de façon endogène.

L'invention permet aussi d'obtenir une plante ou partie de plante, caractérisée en ce qu'elle contient l'ADN recombinant défini précédemment et en ce qu'elle a été sélectionnée par visualisation de l'activité enzymatique de la protéine à activité productrice de H₂O₂ par utilisation d'un test colorimétrique à la peroxydase, appliqué aux racines issues de culture in vitro ou aux tissus végétaux prélevés sur les plantes entières. Les plantes transgéniques sont régénérées directement à partir des racines ou organes sélectionnés par le test colorimétrique à la peroxydase.

Les parties de plantes définies selon l'invention comprennent notamment les hypocotyles, hampes de fleurs, pétioles et préférentiellement les cotylédons. Par partie de plantes, on entend également les cellules cultivées in vitro ou les cellules de ladite plante.

Selon un autre aspect de l'invention, la séquence d'ADN recombinant codant pour une protéine à activité productrice de H₂O₂ est utilisée pour sélectionner des cellules végétales transformées avec une séquence d'intérêt.

Ainsi l'invention concerne également un procédé pour l'obtention de plantes transgéniques exprimant un gène d'intérêt, autre que celui de la protéine à activité productrice de H₂O₂, associé au gène codant pour une protéine productrice de H₂O₂, qui comprend les étapes suivantes de :
(a) transformation de cellules végétales par *Agrobacterium rhizogenes* contenant un ADN recombinant comprenant à la fois un gène codant la protéine productrice de H₂O₂ et un gène codant pour une protéine d'intérêt dans un contexte permettant leur expression dans le végétal ;
(b) sélection des transformants qui contiennent le gène codant pour la protéine d'intérêt par un test colorimétrique à la peroxydase ;
(c) régénération de plantes à partir des transformants sélectionnés et contrôle de l'expression des plantules obtenues par un test colorimétrique à la peroxydase ;
(d) tri selon le phénotype et éventuellement analyse moléculaire de la descendance des plantes transgéniques pour sélectionner ou confirmer les plantes contenant seulement le transgène et non l'ADN-T propre à *A*. *rhizogenes.*
(e) purification, le cas échéant de la protéine d'intérêt produite.

Ce nouveau système de révélation immédiat sur racine est particulièrement intéressant dans le cas où le gène d'intérêt est exprimé à un stade tardif du développement ou dans autre organe végétatif que celui sur lequel est effectué la sélection ou encore s'il est difficile à mettre en évidence. Il est par exemple utilisable pour sélectionner des événements de transformation portant sur un gène d'intérêt placé sous contrôle d'un promoteur spécifique de la graine ou spécifiquement exprimé dans les tissus verts.

La séquence d'intérêt peut être toute séquence d'ADN d'intérêt agronomique ou industriel. Par exemple, elle peut être impliquée dans une voie métabolique donnée, notamment celle des huiles, des amidons, des protéines, des acides aminés, de la lignine, des composants de la paroi cellulaire ou encore dans la voie de résistance aux pathogènes. Cette séquence d'intérêt peut encore être utilisée en sens ou en antisens.

Elle peut également être, par exemple, une séquence régulatrice avantageuse. Elle peut être aussi une séquence codant pour une protéine d'intérêt ou pour un précurseur de cette dernière. A titre d'exemple de séquences codant pour une protéine d'intérêt, on peut citer les séquences codant pour les lipases.

Selon un mode de réalisation préféré de l'invention, la séquence d'intérêt confère aux plantes une résistance aux agents pathogènes, tels que les champignons, les bactéries, ainsi que les arthropodes, notamment les insectes, et les nématodes.

Une telle séquence d'intérêt peut être, par exemple, une séquence codant pour une protéine à activité endochitinase ou pour un précurseur de cette dernière. On sait en effet, comme décrit dans la demande de brevet WO 92/01792, qu'une telle protéine a un effet phytoprotecteur car elle est capable de dégrader la chitine, polymère polysaccharidique constitué d'unités N-acétyl-glucosamine associées par des liaisons β-1,4, qui est un composé structural important de la paroi de la plupart des champignons pathogènes, de l'exosquelette des arthropodes, en particulier des insectes, et de l'enveloppe externe des oeufs et des cystes de nématodes.

Une séquence intéressante codant pour une protéine à activité endochitinase ou pour un précurseur de cette dernière, est celle décrite dans la demande de brevet WO 92/01792, incorporée par référence.

Une autre séquence codant pour une protéine à activité endochitinase ou pour un précurseur de cette dernière est celle de la chitinase d*'Aphanocladium album* décrite dans la demande de brevet EP-Al-531 218, incorporée par référence.

L'invention permet aussi d'obtenir des cellules végétales, caractérisées en ce qu'elles sont transformées par l'ADN recombinant défini précédemment, à savoir l'ADN recombinant comprenant un gène codant pour une protéine à activité productrice de H₂O₂ et un gène codant pour une protéine d'intérêt ainsi que les moyens nécessaires à leur expression. Lesdits moyens sont les mêmes que ceux définis précédemment. Ces cellules végétales peuvent provenir des espèces de grande culture ou des espèces potagères, précédemment indiquées.

L'invention permet aussi d'obtenir une plante ou partie de plante, caractérisée en ce qu'elle exprime la protéine d'intérêt sus-décrite, avec les moyens nécessaires à l'expression du gène codant pour une protéine capable de produire H₂O₂ et notamment l'oxalate oxydase. Cette plante ou partie de plante est sélectionnée par un test colorimétrique de révélation de H₂O₂ formé sur les racines issues de culture in vitro ou de plantes en serre. Les plantes transgéniques sont régénérées directement à partir des racines exprimant le transgène qui sont sélectionnées par le test colorimétrique.

Les parties de plantes sont telles que définies précédemment.

L'invention sera illustrée à l'aide des exemples décrits ci-après.

Dans cette partie expérimentale, on utilise le clone gf-2.8 de la germine de blé décrite par Lane et al., J. Biol. Chem., (1991), 226, 10461.

Une grande partie de l'ensemble des techniques ci-après, bien connues de l'homme du métier, est exposée en détail dans les ouvrages de Sambrook et al. : « Molecular Cloning : a Laboratory Manual », publié en 1989 par les éditions Cold Spring Harbor Press à New York (2^{ème} édition), et dans l'ouvrage de Gelvin et al. : « Plant Molecular Biology Manual », publié en 1988 par les éditions Kluwer Academics.

### EXEMPLES

### Exemple 1 : Transformation de plantes par le gène de la germine de blé et sélection des transformants par un test colorimétrique sur racine

### 1-1 Obtention de colza transgénique

### a) Vecteurs de transformation

Selon un premier mode de réalisation de l'invention, la séquence codant pour la germine de blé est liguée à la séquence promotrice 35S du virus de la mosaïque du chou-fleur et à la séquence terminatrice NOS d'*Agrobacterium tumefaciens*, avant d'être insérée dans le vecteur binaire pBIN19 (Bevan, Nucl. Acid, Res., (1984), 12, 8711-8721) selon le mode opératoire décrit à la Section 2 de la demande WO94/13790, incorporée par référence. Le vecteur obtenu, nommé pPH100, est cloné dans la souche *E. coli* HB101 (Clontech).

Selon un autre mode de réalisation de l'invention, on a opéré de la même manière que ci-dessus en utilisant la séquence promotrice du gène codant pour le promoteur du Facteur d'Elongation EF-1α isolé d*'Arabidopsis thaliana* (Axelos et al, Plant Mol Biol, (1989), 219 :1-2,106) à la place de la séquence promotrice 35S. Le nouveau vecteur, nommé p631, est cloné dans la souche *E*. *coli* HB 101.

La séquence promotrice peut aussi être la séquence promotrice inductible décrite dans la demande de brevet WO94/21793 et portée par le vecteur pPH 118 décrit à l'exemple 3 ci-après.

### b) Transfert dans Agrobacterium rhizogenes

Le transfert des plasmides sus-décrits est réalisé selon la méthode de congélation-décongélation décrite par Gelvin et al. dans Plant Molecular Biology Manual susmentionné, avec la souche *Agrobacterium rhizogenes* A4 décrite par Guerche et al. dans Mol. Gen. Genet., (1987), 206, 382.

Les bactéries sont étalées sur des boîtes de Pétri contenant 100 mg/l de rifampicine et 25 mg/l de kanamycine. Les colonies qui se forment alors sont repiquées plusieurs fois sur le milieu de sélection. La présence du gène d'oxalate oxydase dans *Agrobacterium rhizogenes* est vérifiée par la méthode de Southern, sur une préparation d'ADN total (lyse des bactéries, purification de l'ADN par extraction à l'aide du mélange phénol/chloroforme selon le protocole décrit par Gelvin dans l'ouvrage cité ci-dessus, coupure de l'ADN purifié à l'aide d'enzymes de restriction, électrophorèse sur gel d'agarose, transfert sur membrane et hybridation selon les techniques bien connues de l'homme du métier.

### c) Obtention de racines transformées et sélection

La transformation est réalisée selon le protocole de Guerche et al (1987) et les différents milieux de culture, dont la composition est présentée dans le tableau 1 ci-après, sont ceux décrits par Pelletier et al. (Mol. Gen. Genet., (1983) 191,244).

Des segments de tige sont prélevés sur l'extrémité apicale de plantes de colza (*Brassica napus :* variétés de printemps Brutor et variétés d'hiver Nickel ou Navajo). Ces segments sont stérilisés en surface, rincés dans de l'eau stérile, découpés en segments de 1,5 cm de long environ et placés dans un tube contenant le milieu *A*. Alternativement, l'explant utilisé pour la transformation peut être constitué par des cotylédons prélevés sur de jeunes germinations de huit jours environ. L'inoculation de l'extrémité des segments de hampes, des hypocotyles ou des pétioles de cotylédons est effectuée par dépôt d'une suspension de la souche *d'Agrobacterium rhizogenes* contenant le vecteur pPH100 ou le vecteur p631. Des racines transformées apparaissent sur le segment de tige ou sur les pétioles de cotylédons au bout de 1 à 2 semaines, elles sont prélevées au bout de 3 semaines et placées sur le milieu *B* gélosé (15 g/l). Ces racines contiennent l'ADN-T du vecteur pRi propre à *Agrobacterium rhizogenes* seul ou accompagné de l'ADN-T du vecteur porteur du gène codant pour l'oxalate oxydase.

La sélection des transformants contenant l'ADN-T des plasmides pPH 1 00 ou p631 porteurs du gène codant pour la germine de blé, est réalisée selon le protocole suivant :
- la première étape consiste à prélever au scalpel les extrémités apicales (0,5 à 1cm) de racines cultivées in vitro de préférence (stade décontamination ou culture liquide) ou de fragments de feuilles de plantes cultivées en serre, et les introduire dans des tubes Eppendorf de 1,5 ml contenant 0,5 ml de mélange réactionnel « oxalate oxydase » (oxalate de Na 0,015 M dans un tampon succinate pH 4 ; 0,05 M). Les tubes contenant les racines sont conservées dans la glace jusqu'à la fin du prélèvement.
- la seconde étape est l'incubation des tubes au bain-marie à 37°C pendant 1 heure, conditions permettant le déroulement de la réaction catalysée par l'oxalate oxydase.
- la troisième et dernière étape est la révélation de l'eau oxygénée produite dans l'étape précédente : sont ajoutés successivement au mélange réactionnel un substrat dont l'oxydation s'accompagne d'un changement de coloration ; ce substrat est choisi parmi les composés phénoliques ou les amines aromatiques et, par exemple, 0,1 ml de 4-chloro-1-naphtol à 0,3 % dans l'éthanol absolu et 0,1 ml de peroxydase à 0,006 % dans le tampon Tris pH 7,6, 50 mM. Les tubes sont agités puis incubés au moins 2 heures au bain-marie à 37°C. Une coloration bleue est obtenue sur les racines transformées, préférentiellement au niveau de la partie apicale et du cylindre central des racines. Ce test coloré sur échantillon de racine permet la détection des transformants qui ont intégré et expriment le gène d'oxalate oxydase.

Les solutions des produits utilisés sont décrites ci-après :
Tampon Succinate 0,05M, pH4, QSP 250 ml (conserver à 4°C, 3 semaines maximum)
   3,375 g de Succinate de Na, ajuster à pH4 avec HCl
Mélange réactionnel « oxalate-oxydase » (préparer extemporanément)
   18 mg d'oxalate de Na dans 10 ml de Tampon Succinate
Solution mère de 4-Chloro-1-Naphtol (conserver à 4°C)
   0,3 g/10 ml éthanol absolu
Solution d'utilisation de 4-Chloro-1-Naphtol (préparer extemporanément)
   0,1 ml solution mère/10 ml Tris 50 mM ; pH 7,6
Tampon Tris 50 mM pH 7,6 QSP 250 ml (conserver à 4°C)
   1,96 g Tris-HCI, ajuster à pH 7,6 avec NaOH
Peroxydase (aliquoter et congeler à -20°C)
   6 mg/100 ml tampon Tris 50 mM ; pH 7,6

### d) Régénération de plantes transformées et tri phénotypique

Des fragments de racines sélectionnées sont incubés pendant 15 jours sur le milieu *D* contenant 3 mg/l d'acide 2,4-dichlorophénoxyacétique, puis placés sur un milieu d'induction RCC de bourgeons. Les plantes racinées sont ensuites obtenues par passages des bourgeons sur les milieux F et G. Les plantes sont cultivées et auto fécondées pour donner des graines. L'expression des plantes régénérées obtenues peut être facilement contrôlée par des techniques simples (squash, empreinte, dot ou coloration directe sur des fragments de feuilles, de tiges, ...). Un tri de la descendance selon les caractères phénotypiques des plantes transformées permet d'éliminer les plantes contenant l'ADN-T de pRi, qui présentent notamment des feuilles gaufrées et des entre-noeuds raccourcis. Ce tri est ensuite confirmé par des méthodes d'analyse moléculaire.

### 1-2 Obtention de chou-fleur transgénique

Selon le même protocole, des segments de tige sont prélevés sur de jeunes fleurs de *Brassica oleracea* L. var. *Botrytis* cv. Taroke et transformées via les souches d*'Agrobacterium rhizogenes* A4 précitées. Les racines exprimant le gène codant pour l'oxalate oxydase sont sélectionnées selon le test coloré décrit et mises à régénérer pour l'obtention de plantes transgéniques. Les plantes régénérées obtenues sont cultivées en serre, vemalisées puis auto fécondées et leur descendance triée sur la base de caractères phénotypiques et d'analyses moléculaires pour ne conserver que les plantes qui contiennent uniquement l'ADN-T porteur du gène d'oxalate oxydase.

### 1-3 Obtention de cals de tournesol transgéniques

Des segments de pétioles sont prélevés sur des plantes de tournesol *Helianthus annuus* (Variété Euroflor Rustica Prograin Génétique) âgées de 6 à 10 semaines. Les segments sont désinfectés par trempage pendant 30 minutes dans une solution d'hypochlorite de calcium à 1%. Les segments de pétioles sont ensuite placés dans un tube contenant du milieu de culture de Murashige et Skoog gélosé. L'inoculation de l'extrémité de ces segments est effectuée par dépôt d'une suspension de la souche d*'Agrobacterium rhizogenes* contenant le plasmide pPH 100. Des racines transformées apparaissent ensuite sur le segment de pétiole au bout de 1 mois environ. Ces racines sont prélevées et placées sur le milieu M gélosé (Tableau 2, en annexe) additionné de 6g/l d'agarose.

Une sélection des racines exprimant la protéine à activité oxalate oxydase est effectuée au stade racine selon le protocole précédemment décrit. Les racines positives au test colorimétrique, sont alors repiquées sur le même milieu, donnant des cals entièrement transformés qui expriment l'oxalate oxydase.

### 1-4 Obtention de tabac transgénique

La nervure centrale de feuilles de tabac est découpée en fragments qui sont placés sur un milieu de Murashige et Skoog (MS) gélosé. Ces explants sont inoculés à l'aide d'une suspension de la souche *A. rhizogenes* contenant le plasmide pPH100. Les racines qui apparaissent au bout de quelques jours sont repiquées sur le même milieu contenant 500 mg/l de cefotaxime afin d'éliminer la bactérie et transférées ensuite sur un milieu gélosé de Murashige et Skoog contenant 0,1 mg/l d'AIA (acide 3-indolyl acétique) et 1 mg/l de BAP (6-benzyl aminopurine). Les bourgeons qui se forment sont repiqués sur un milieu MS gélosé et les plantes obtenues transférées en serre.

### 1-5 Obtention de tomate transgénique

Des plantes de tomate peuvent être obtenues selon le même protocole de transformation et sélection, à partir de racines de plantes transformées (Shahin et al, TAG (1986) 72 :770 ; Morgan et al, Plant Science (1987), 49 :37).

### Exemple 2 : Utilisation de ce procédé de sélection à la peroxydase pour obtenir des plantes transgéniques exprimant un second gène d'intérêt: le gène codant pour une protéine à activité endochitinase.

### 2-1 Obtention de colza transgénique

Le vecteur de transformation pPH 106, comprenant le gène codant pour la germine de blé issu du plasmide pPH100 sus-décrit et le gène chimérique codant pour une activité endochitinase (WO 92/01792), est obtenu selon le procédé décrit dans la demande de brevet WO 94/13790, incorporée par référence.

La préparation du vecteur de transformation comprend les étapes suivantes :

### a) préparation du fragment portant le gène codant pour l'oxalate oxydase

Le fragment HindIII-EcoRI de 1420 pb environ du plasmide pPH100 cité dans la section 1-1 de l'exemple 1 est purifié et recloné dans un vecteur pUC19 selon les méthodes bien connues de l'homme du métier. Ce plasmide est ensuite linéarisé grâce à l'endonucléase de restriction EcoRI et l'extrémité cohésive est remplie au moyen du fragment de Klenow. Puis, après coupure par l'endonucléase HindIII, le fragment HindIII- extrémité franche est purifié.

### b) préparation du fragment portant un gène hybride codant pour une protéine à activité endochitinase

Le fragment HindIII-EcoRI provenant du plasmide pBR1 décrit dans la demande de brevet WO 92/01792 exemple 1, incorporée par référence, et contenant un gène chimérique codant pour une protéine à activité endochitinase qui comprend le promoteur 35S, une séquence codant pour une chitinase hybride tomate-tabac et le terminateur NOS est purifié, recloné dans le vecteur pUC19, puis le site HindIII est détruit d'une manière classique. Le fragment extrémité franche- EcoR1 est purifié.

### c) préparation d'un vecteur de transformation des plantes ne comportant pas de gène de résistance à la kanamycine

Le fragment NheI-HindIII comprenant la partie codant pour le gène de résistance à la kanamycine est éliminé de l'ADN-T du plasmide pBIN19. L'utilisation, selon les méthodes bien connues de l'homme du métier, des oligonucléotides CTAGCA et AGCTTG, permet de recirculariser le plasmide en recréant les sites de restriction NheI et HindIII. Le plasmide résultant est ensuite linéarisé par les endonucléases de restriction HindIII et EcoRI.

### d) assemblage du vecteur de transformation

On a ligué à l'aide de l'ADN ligase T4 le gène codant pour l'oxalate oxydase obtenu en a) ci-dessus et le gène chimérique codant pour une protéine à activité chitinase (obtenu en b) ci-dessus) dans un vecteur binaire pBIN19 dans lequel on a éliminé le gène de résistance à la kanamycine s'exprimant dans les plantes (obtenu en c) ci-dessus). Le vecteur obtenu, appelé pPH106, est cloné dans la souche E. Coli HB101 (Clontech).

Les étapes de transformation, sélection et régénération sont réalisées selon le protocole fourni dans le premier exemple.

### 2-2 Mise en évidence de l'expression de la protéine d'intérêt dans les plantes transgéniques régénérées

Ainsi qu'il est décrit dans la demande de brevet WO 94/13790, incorporée par référence, des techniques de Western blot et de mesure de l'activité chitinolytique sur des extraits bruts de protéines de plantes transformées, permettent de confirmer l'expression corrélée de l'endochitinase avec celle de l'oxalate oxydase.

La préparation des extraits bruts de protéines de plantes transformées est effectuée selon la méthode suivante : les fragments de tissus (cals et feuilles de plantes) ont été congelés dans l'azote liquide, réduits en poudre et stockés à -20°C.

Pour la réalisation d'électrophorèses, l'oxalate oxydase est extraite directement à partir de la poudre végétale par le tampon de charge de Laemmli (réf. ci-après).

Pour les dosages d'activité oxalate oxydase, l'extrait enzymatique est réalisé par mise en suspension de la poudre végétale dans un tampon succinate 0,05 M, pH 4.

Pour les dosages de protéines, l'extrait végétal, en suspension dans le tampon succinate ci-dessus, est centrifugé à 10 000 g pendant 5 min.

La concentration des protéines totales est déterminée sur les surnageants, appelés ci-après les extraits bruts de protéines, en suivant la technique de Bradford, Anal. Biochem., (1976), 72, 248-254).

Les expériences de Western blot ou de mise en évidence de l'activité chitinolytique suivent les protocoles suivants :

### a) Immunodétection de la chitinase hybride (Western blot)

On soumet les extraits bruts de protéines à un Western blot, technique bien connue de l'homme du métier et décrite par Towbin et al. (Proc. Ntl. Acad. Sci. USA, (1979), 76, 4350-4354), qui comprend notamment les étapes suivantes :
- dénaturation par chauffage à 100°C pendant 10 min dans un tampon, dénommé tampon de charge, constitué de Tris 0,125M, pH6,8, SDS 4%, bleu de bromophénol 0,002%, glycérol 20%, β-mercaptoéthanol 10% (selon le protocole décrit par Laemmli U.K, Nature, (1970), 227, 680-685), suivie d'une centrifugation à 10 000g ;
- séparation électrophorétique des différentes protéines contenues dans le solubilisat selon le protocole décrit par Laemmli ;
- électrotransfert desdites protéines contenues dans le gel sur une membrane en PVDF (selon la technique de Towbin et al., réf. ci-dessus).

L'immunodétection est réalisée selon le protocole qui comprend les étapes suivantes :
- saturation de la membrane PVDF (fluorure de polyvinylidène) sur laquelle les protéines ont été transférées par incubation pendant au minimum 2 h à 37 °C dans une solution de gélatine à 3% dans du tampon phosphate salin contenant 0,05 % de détergent Tween 20.
- incubation (pendant 1 h à 37 °C) en présence de l'immunsérum préparé précédemment (contenant les anticorps polyclonaux reconnaissant la protéine recombinante), dilué au 1/10 000 dans du tampon phosphate salin.
- 3 lavages dans du tampon phosphate salin contenant 0,05% de détergent Tween 20.

L'immunodétection de la protéine d'intérêt est réalisée grâce à un immunsérum contenant des anticorps polyclonaux reconnaissant la protéine hybride à activité chitinase (cf. WO 92/01792 exemple 5, incorporé par référence).

Le complexe antigène-anticorps est ensuite révélé à l'aide d'un système streptavidine-biotine conjugué à la phosphatase alcaline avec le kit RPN 23 d'Amersham (« Blotting détection kit »), utilisé selon les indications du fabricant.

L'empreinte obtenue montre, pour les feuilles de plantes de tabac transformées par le plasmide pPH106, la présence d'une protéine de poids moléculaire apparent d'environ 26 ± 6 kDa reconnue par les anticorps polyclonaux et absente des feuilles des plantes témoins. Cette protéine a le même poids moléculaire apparent que la protéine hybride à activité chitinase décrite dans la demande WO 92/01792.

### b) Mise en évidence de l'activité chitinolytique de la protéine recombinante.

L'activité chitinolytique des extraits bruts de protéines de feuilles de plantes transformées par le plasmide pPH106 et d'extrait brut de protéines de feuilles de plantes non transformées, peut être mesurée selon la méthode suivante.

L'activité endochitinase de la protéine est mesurée par une méthode radiochimique permettant d'estimer la quantité de monomères ou d'oligomères libérés par l'enzyme à partir d'un substrat (la chitine tritiée). Cette méthode, décrite par Molano et al. Anal. Biochem., (1977), 83, 648-656), est résumée ci-après.

A un volume d'extrait protéique de 10 µl sont ajoutés 50 µl d'une suspension de chitine tritiée d'activité spécifique 0,58 MBq/ml. Le volume final est ajusté à 300 µl avec du tampon d'acétate de sodium 0,2 M de pH 5,0. Après 90 min d'incubation à 30°C, la réaction d'hydrolyse de la chitine est arrêtée par 100µl d'acide trichloracétique à 20%. Les tubes réactionnels sont ensuite centrifugés pendant 10 min à 12 000 g. Une partie aliquote de 100 µl de surnageant renfermant les oligomères solubles de chitine est prélevée et la radioactivité correspondante est mesurée par scintillation liquide en présence de 5 ml de mélange scintillant. On exprime l'activité chitinolytique spécifique en dpm/µg de protéine.

On constate que les extraits de plantes transformés par le plasmide pPH106 ont une activité chitinolytique significativement supérieure à celle de l'extrait de plantes témoin la sélection par test colorimétrique permet donc d'obtenir des plantes exprimant un gène d'intérêt, en l'occurrence le gène hybride codant pour une protéine à activité chitinase décrit dans la demande de brevet WO 92/01792.

Une variante de l'ADN recombinant sus-décrit peut être obtenue en substituant le promoteur 35S du virus de la mosaïque de chou-fleur par le promoteur du gène EF-1α d'*Arabidopsis*, en amont de l'un ou des deux gènes précédemment décrits.

Selon le même procédé, des plantes de chou-fleur, tournesol, tabac et tomate transgéniques peuvent être obtenues.

### Exemple 3 : Utilisation et induction d'un promoteur inductible

Il est possible d'utiliser préférentiellement un promoteur inductible dans des situations de stress (par exemple choc thermique, blessure, choc hormonal, éliciteur biotique ou abiotique ou infection bactérienne, fongique ou virale), qui s'exprime à un niveau soutenu dans les différents organes et tissus d'un végétal, notamment les racines et la méristème d'une plante.

Dans cet exemple, on a utilisé le promoteur inductible au stress qui comprend la séquence d'ADN (séquence B précédée de la séquence C) [SEQ ID N° 4 précédée de SEQ ID N°5] ou une séquence présentant un degré d'homologie élevé avec cette séquence, telle que décrite dans la demande WO94/21793 incorporée par référence. Ce promoteur est dénommé ci-après p246-C.

### 3-1 Préparation des vecteurs de transformation

### a) Construction promoteur inductible-oxalate oxydase

Le vecteur pPH100 décrit dans l'exemple 1-1 a) est ouvert à l'aide des enzymes de restriction HindIII et BamHI, puis le fragment de 13500 paires de bases environ correspondant à la partie codante de l'oxalate oxydase suivie du terminateur NOS dans le vecteur pBIN19, est purifié par électrophorèse sur gel d'agarose.

Le vecteur pPH118 obtenu par insertion, selon les procédés bien connus de l'homme du métier, du promoteur inductible au stress p246-C défini ci-dessus dans le plasmide pTZ19R commercialisé par PHARMACIA, a été ouvert à l'aide des mêmes enzymes de restriction. Après une électrophorèse sur gel d'agarose, le fragment HindIII-BamHI de 1275 pb environ est isolé et purifié. Il correspond à la séquence B précédée de la séquence C du promoteur inductible telle que décrites ci-dessus et dans la demande WO94/21793.

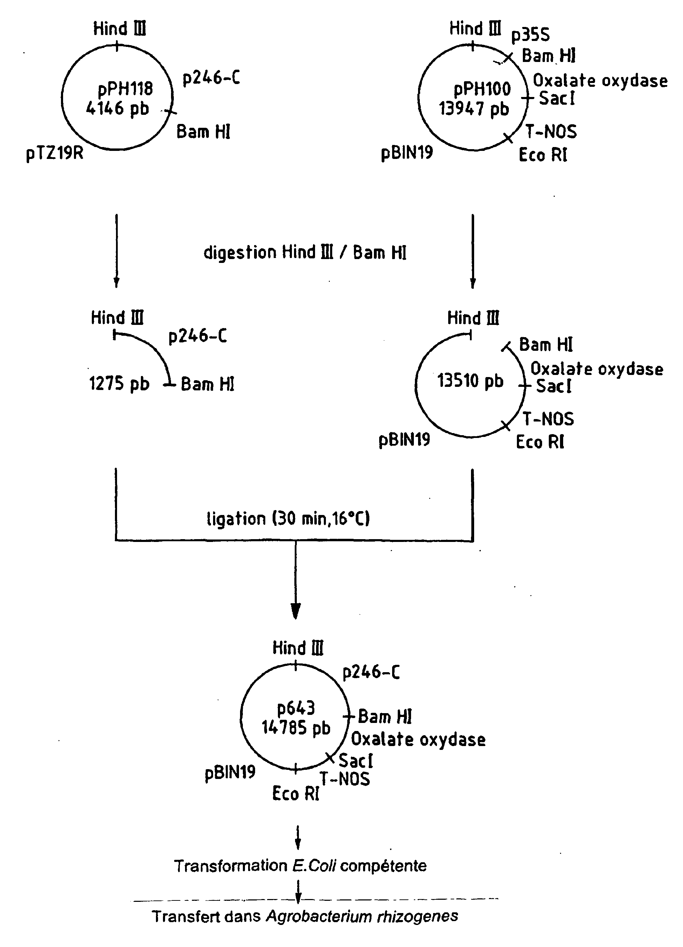

La ligation de la séquence promotrice ainsi obtenue au vecteur HindIII-BamHI ci-dessus met la partie codante de l'oxalate oxydase sous le contrôle du promoteur inductible et du terminateur NOS ; le vecteur binaire ainsi créé est appelé p643. La construction de ce vecteur est illustrée sur la figure I annexée.

### 3-2 Transformation et sélection

La transformation du colza est réalisée selon le protocole décrit ci-dessus à l'exemple 1-1. Les racines transformées exprimant le gène d'oxalate oxydase sont sélectionnées comme décrit précédemment et mises à régénérer. Après un choc hormonal, un cal se forme à partir des racines et produit des bourgeons qui sont ensuite repiqués pour donner des plantes. Ces plantes sont transférées en serre après une période d'acclimatation.

Selon ce même protocole des plantes de tabac, tomate et chou-fleur peuvent être obtenues.

### Exemple 4 : Sélection des transformants par un test colorimétrique sur milieu liquide ou empreinte de boîte de milieu gélosé ou membrane.

L'oxalate oxydase pouvant diffuser à partir du lieu où elle est exprimée, il est possible d'utiliser d'autres supports que la racine pour réaliser le test colorimétrique.

### 4-1. Milieu liquide

La sélection des transfonnants porteurs du gène codant pour la germine de blé peut être réalisée directement sur le milieu de culture in vitro après élimination des agrobactéries. Après avoir ôté les jeunes plantes décrites au point c) de l'exemple 1 § 1.1, on ajoute au milieu de culture ou à un échantillon prélevé de celui-ci, le mélange réactionnel "oxalate oxydase" puis l'on procède comme décrit à l'exemple 1 pour favoriser la réaction enzymatique et révéler les produits ainsi obtenus.

### 4-2. Empreinte sur membrane

### a) Tri de jeunes plantes

Des graines de colza transgéniques obtenues selon l'exemple 1, §1.1, sont stérilisées en surface et mises à germer dans une boîte de matière plastique dont le fond est recouvert de plusieurs épaisseurs de papier filtre et d'une membrane en PVDF, Hybond C ou nitrate de cellulose, classiquement utilisées pour la réalisation de Western blots. Les graines sont disposées sur la membrane, sur une grille permettant de repérer leur position. La membrane est mouillée à l'aide d'eau stérile ; la boîte est ensuite fermée et placée dans une enceinte de culture pendant huit jours environ. Lorsque les racines des jeunes germinations sont longues de 1 à 2 cm, les plantules sont transférées dans une autre boîte, sur un papier filtre mouillé, en respectant la position qu'elles avaient sur la membrane.

La membrane est ensuite révélée selon une variante du protocole décrit à l'exemple 1 §1.1 :
- la membrane est mise à incuber à 37°C pendant une heure, après avoir été imbibée du mélange réactionnel « oxalate oxydase » (oxalate de Na 0,015 M dans un tampon succinate pH 4, 0,05M),
- la membrane est révélée par l'addition du substrat (composé phénolique ou amine aromatique) et par exemple de 4-chloro-1-naphtol à 0,03% dans l'éthanol absolu et une solution de peroxydase à 0,0006 % dans le tampon Tris-HCl pH 7,6 ; 50 mM. Cette révélation est poursuivie 2 heures à 37°C.
Les empreintes sur la membrane de racines de plantes exprimant l'oxalate oxydase se colorent en bleu, ce qui permet de repérer les transformants à conserver.

### b) Tri sur empreintes réalisées à partir de coupes de tissus

Différents organes de plantes (feuilles, pétioles, racines, pièces florales...) transformées selon les étapes décrites à l'exemple 1, sont prélevés. Sur ces échantillons frais, une section est réalisée à l'aide d'un scalpel et la surface de coupe est fermement appliquée contre la surface d'une membrane de nitrocellulose. Après un séchage à l'air de quelques minutes, la révélation de la membrane est effectuée comme décrit ci-dessus.

### Exemple 5 : Utilisation d'un promoteur spécifique de la graine.

Le système de révélation immédiat sur racine est particulièrement intéressant dans le cas où le gène d'intérêt est exprimé à un stade tardif du développement, comme dans la graine par exemple.

### 5-1 Préparation des vecteurs de transformation

Un premier vecteur contenant une séquence nucléique codant pour une lipase sous contrôle du promoteur PGEA1 *d*'*Arabidopsis thaliana*, spécifique de la graine (Gaubier et al., 1993 cité précédemment) a été obtenu selon les méthodes de clonage connues de l'homme du métier à partir des séquences dont les informations sont disponibles dans Genbank : la séquence U02622 de 1641 nucléotides codant pour une lipase 1 de *Geotrichum candidum-* souche ATCC 34614 a été placée sous contrôle de la séquence nucléotidique promotrice PGEA1 d'*Arabidopsis thaliana* (Z11158, 1659 nucléotides) et fusionnée au terminateur Nos, dans un plasmide pBluescript.

Comme décrit au point d) de l'exemple 2 § 2.1, le fragment obtenu ci-dessus a été ligué à l'aide de l'ADN ligase T4 au gène codant pour l'oxalate oxydase dans un vecteur binaire pBIN19 pour l'étape de transformation.

### 5-2 Transformation et sélection

La transformation du colza est réalisée selon le protocole décrit à l'exemple 1 à l'aide du vecteur dérivé de pBIN19 décrit ci-dessus et la sélection des transformants est réalisée dès la formation des racines, selon l'une des méthodes décrites à l'exemple 1 ou l'exemple 4.

Les résultats montrent qu'il est possible, selon le système de révélation immédiate au stade racine de l'invention, de sélectionner très tôt des transformants qui expriment normalement le gène d'intérêt à un stade tardif du développement (graine) ou dans organe végétatif autre que celui sur lequel la sélection a été réalisée.

Des plantes de tabac, tomate et chou-fleur peuvent également être obtenues selon un protocole semblable.

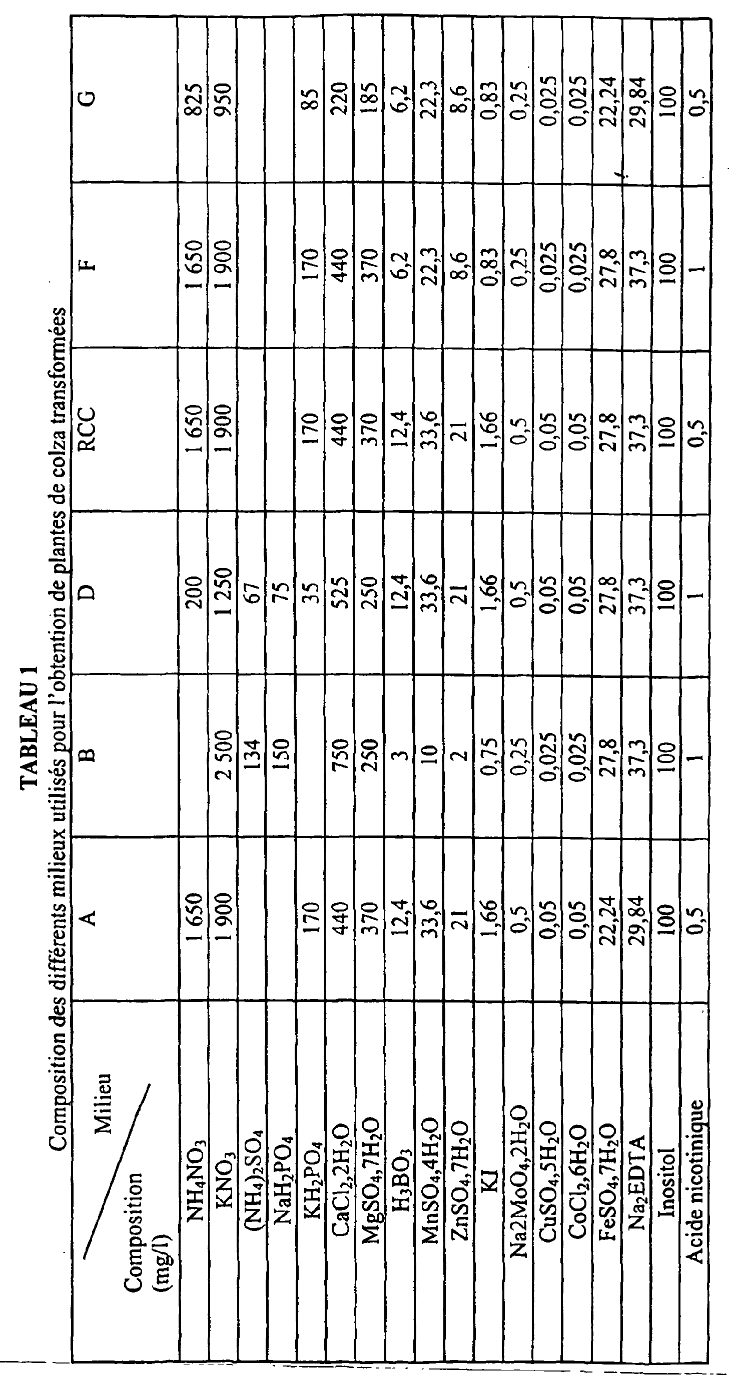

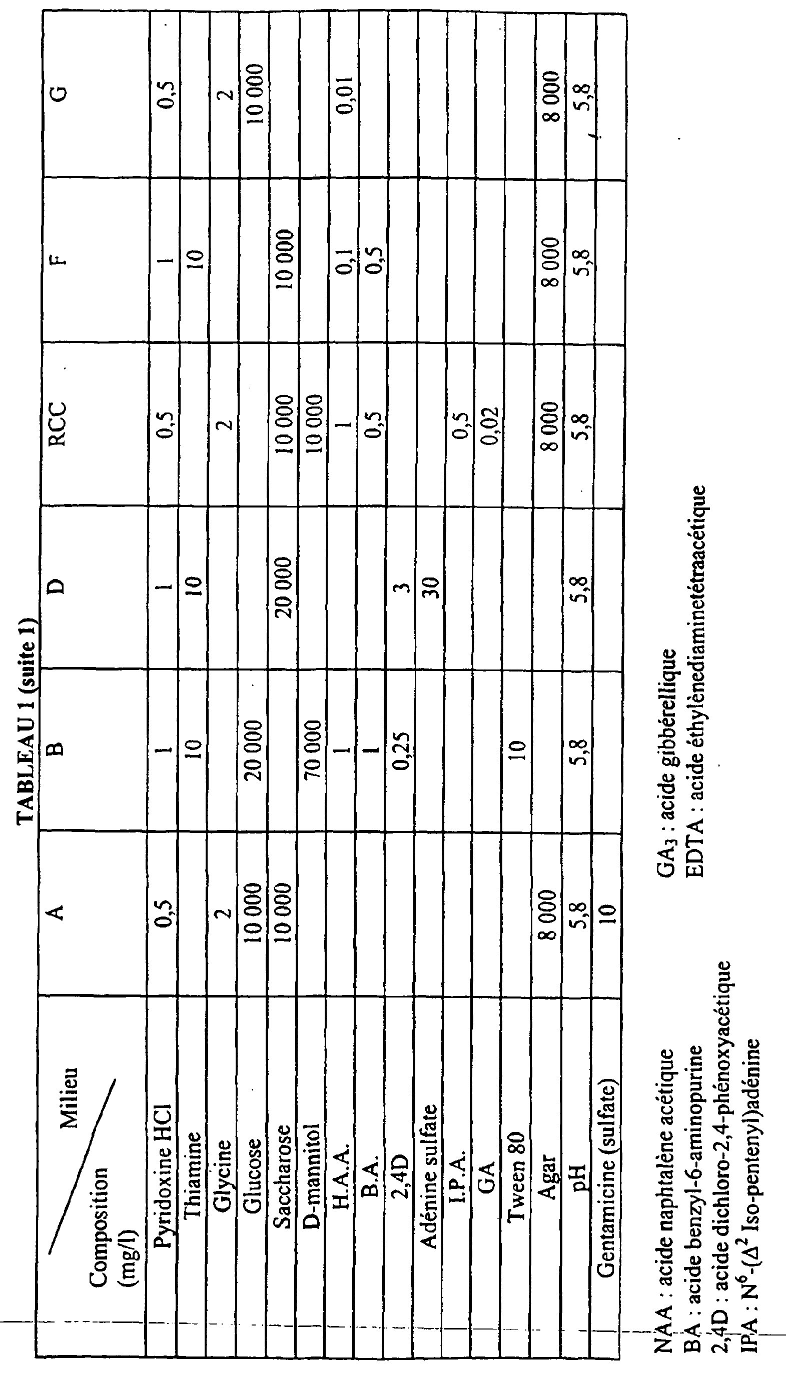

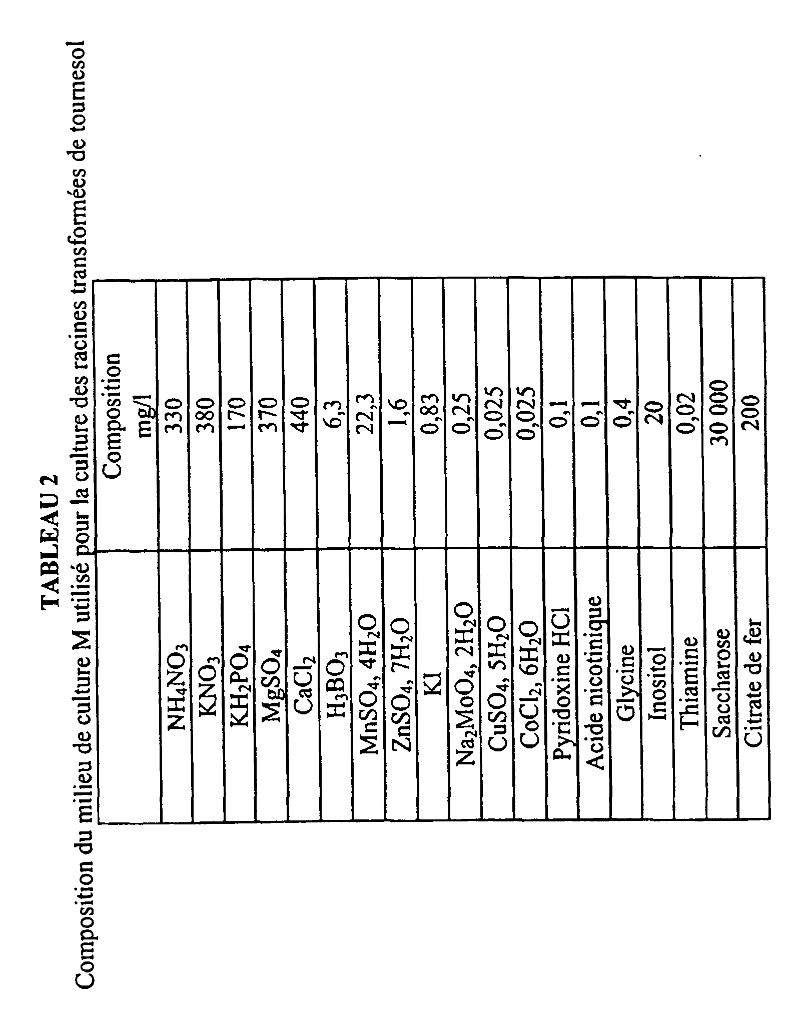

## Revendications

1. Procédé d'obtention de plantes transgéniques exprimant une protéine à activité productrice de H₂O₂, **caractérisé en ce qu'**il comprend les étapes suivantes de :
(a) transformation de cellules végétales ou explants par *Agrobacterium rhizogenes* contenant un vecteur porteur d'un gène codant pour une protéine productrice de H₂O₂ dans un contexte qui permet son expression dans le végétal, ladite transformation induisant la formation de racines transformées ;
(b) sélection des racines transformées qui contiennent et expriment ce gène par un test colorimétrique à la peroxydase ;
(c) régénération des plantules à partir des racines sélectionnées et contrôle de l'expression dudit gène codant pour une protéine productrice de H₂O₂ dans les plantules obtenues, ledit contrôle d'expression étant réalisé par un test calorimétrique à la peroxydase ;
(d) tri phénotypique et éventuellement analyse moléculaire de la descendance des plantes transgéniques, permettant la sélection ou la confirmation des plantes transgéniques obtenues contenant seulement le transgène et non l'ADN-T propre à *A*. *rhizogenes*.

2. Procédé d'obtention de plantes transgéniques exprimant un gène d'intérêt et un gène codant pour une protéine à activité productrice de H₂O₂, **caractérisé en ce qu'**il comprend les étapes suivantes de :
(a) transformation de cellules végétales ou explants par *Agrobacterium rhizogenes* contenant un ADN recombinant comprenant à la fois un gène codant la protéine productrice de H₂O₂ et un gène codant pour une protéine d'intérêt dans un contexte permettant leur expression dans le végétal, ladite transformation induisant la formation de racines transformées ;
(b) sélection des racines transformées qui contiennent le gène codant pour la protéine d'intérêt et expriment le gène codant pour une protéine productrice de H₂O₂ par un test calorimétrique à la peroxydase ;
(c) régénération de plantules à partir des racines sélectionnées et contrôle de l'expression dudit gène codant pour une protéine productrice de H₂O₂ dans les plantules obtenues, ledit contrôle d'expression étant réalisé par un test colorimétrique à la peroxydase ;
(d) tri selon le phénotype et éventuellement analyse moléculaire de la descendance des plantes transgéniques pour sélectionner ou confirmer les plantes contenant seulement le transgène et non l'ADN-T propre à *A. rhizogenes ;*
(e) purification, le cas échéant de la protéine d'intérêt produite.

3. Procédé selon l'une des revendications 1 ou 2, **caractérisé en ce que** le test colorimétrique à la peroxydase selon l'étape (b) ou (c) est effectué en présence d'un substrat pour ladite protéine à activité productrice de H₂O₂ et en présence de peroxydase pour révéler la formation de H₂O₂.

4. Procédé selon l'une des revendications 1 à 3, **caractérisé en ce que** la protéine productrice de H₂O₂ est une protéine à activité oxalate oxydase.

5. Procédé selon la revendication 4 **caractérisé en ce que** la protéine à activité oxalate oxydase est choisie parmi le groupe consistant en l'oxalate oxydase d'orge, de sorgho, de mousse *Mnium menziesii* ou la germine de blé.

6. Procédé selon l'une des revendications 1 à 5, **caractérisé en ce que** le test colorimétrique à l'étape (b) est effectué sur un échantillon de la racine.

7. Procédé selon l'une des revendications 1 à 5, **caractérisé en ce que** le test colorimétrique à l'étape (b) est effectué sur milieu liquide d'incubation après élimination des agrobactéries.

8. Procédé selon l'une quelconque des revendications 1 à 5, **caractérisé en ce que** le test colorimétrique à l'étape (b) est effectué sur l'empreinte laissée par les explants transformés sur un milieu gélosé ou sur une membrane.

9. Procédé selon l'une des revendications 1 à 8, **caractérisé en ce que** la sélection à l'étape (b) est réalisée en présence de l'acide oxalique en tant que substrat de la protéine à activité productrice de H₂O₂.

10. Procédé selon la revendication 9, **caractérisé en ce que** la concentration en acide oxalique est comprise entre 5 et 50 mM.

11. Procédé selon la revendication 10, **caractérisé en ce que** la concentration en acide oxalique est de 15 mM.

12. Procédé selon l'une des revendications 1 à 5, **caractérisé en ce que** le test colorimétrique à l'étape (c) est effectué sur un échantillon de tissu végétal des plantules obtenues.

13. Procédé selon la revendication 2, **caractérisé en ce que** le gène d'intérêt est un gène d'intérêt qui s'exprime à un stade tardif du développement.

14. Procédé selon l'une quelconque des revendications 1 à 13, **caractérisé en ce que** les cellules végétales sont des cellules végétales provenant d'une espèce de grande culture choisie parmi le colza, le chou-fleur, le tournesol, le blé, le maïs, l'orge, le tabac, le soja, ou d'une espèce potagère telle que la tomate, le melon, la laitue, l'oignon.

15. Procédé selon l'une quelconque des revendications 1 à 14, **caractérisé en ce que** les cellules végétales sont des cellules de cotylédons, d'hypocotyles ou de hampes florales.

16. Procédé selon l'une quelconque des revendications 1 à 15, **caractérisé en ce que** les cellules végétales ne produisent pas d'oxalate oxydase de façon endogène.

17. Procédé selon l'une quelconque des revendications 2 à 16, **caractérisé en ce que** la protéine d'intérêt est une endochitinase.

18. Procédé selon l'une quelconque des revendications 2 à 16, **caractérisé en ce que** la protéine d'intérêt est choisie parmi le groupe consistant en une protéine impliquée dans la voie métabolique des huiles, des amidons, des protéines, des acides aminés, de la lignine, des composants de la paroi cellulaire ou dans la voie de résistance aux pathogènes.

19. Procédé selon l'une quelconque des revendications 2 à 16, **caractérisé en ce que** la protéine d'intérêt confère aux plantes une résistance aux agents pathogènes.

## Claims

1. Method for obtaining transgenic plants expressing a protein with H₂O₂ producing activity, **characterized in that** it comprises the following steps of:
(a) transforming plant cells or explants with *Agrobacterium rhizogenes* containing a vector carrying a gene encoding a protein producing H₂O₂ in a context which allows its expression in the plant, the said transformation inducing the formation of transformed roots;
(b) selecting the transformed roots which contain and express this gene, by a peroxidase-based colorimetric test;
(c) regenerating the plantlets from the roots selected and monitoring the expression of the said gene encoding a protein producing H₂O₂ in the plantlets obtained, the said expression monitoring being performed by a peroxidase-based colorimetric test;
(d) sorting according to the phenotype and optionally molecular analysis of the progeny of the transgenic plants, allowing the selection or the confirmation of transgenic plants obtained containing only the transgene and not the T-DNA specific to *A*. *rhizogenes.*

2. Method for obtaining transgenic plants expressing a gene of interest and a gene encoding a protein with H₂O₂ producing activity, **characterized in that** it comprises the following steps of:
(a) transforming plant cells or explants with *Agrobacterium rhizogenes* containing a recombinant DNA comprising both a gene encoding the H₂O₂ producing protein and a gene encoding a protein of interest in a context allowing their expression in the plant, the said transformation inducing the formation of transformed roots;
(b) selecting the transformed roots which contain the gene encoding the protein of interest and express the gene encoding a protein producing H₂O₂ by a peroxidase-based colorimetric test;
(c) regenerating plantlets from the roots selected and monitoring the expression of the said gene encoding a protein producing H₂O₂ in the plantlets obtained, the said expression monitoring being performed by a peroxidase-based colorimetric test;
(d) sorting according to the phenotype and optionally molecular analysis of the progeny of the transgenic plants in order to select or confirm the plants containing only the transgene and not the T-DNA specific to *A*. *rhizogenes,*
(e) purifying, where appropriate, the protein of interest produced.

3. Method according to either one of Claims 1 or 2, **characterized in that** the peroxidose-based colorimetric test according to step (b) or (c) is carried out in the presence of a substrate for the said protein with H₂O₂ producing activity and in the presence of peroxidose in order to reveal the production of H₂O₂.

4. Method according to one of Claims 1 to 3, **characterized in that** the H₂O₂ producing protein is a protein with oxalate oxidase activity.

5. Method according to Claim 4, **characterized in that** the protein with oxalate oxidase activity is selected from the group consisting of the oxalate oxidase from barley, from sorghum, of the moss *Mnium menziesii,* or the wheat germine.

6. Method according to one of Claims 1 to 5, **characterized in that** the colorimetric test in step (b) is carried out on a sample of the root.

7. Method according to one of Claims 1 to 5, **characterized in that** the colorimetric test in step (b) is carried out on a liquid medium for incubation after removing the agrobacteria.

8. Method according to one of Claims 1 to 5, **characterized in that** the colorimetric test in step (b) is carried out on the print left by the transformed explants on an agar medium or on a membrane.

9. Method according to one of Claims 1 to 8, **characterized in that** the selection in step (b) is carried out in the presence of oxalic acid as substrate for the protein with H₂O₂ producing activity.

10. Method according to Claim 9, **characterized in that** the concentration of oxalic acid is comprised between 5 and 50 mM.

11. Method according to Claim 10, **characterized in that** the concentration of oxalic acid is 15 mM.

12. Method according to one of Claims 1 to 5, **characterized in that** the colorimetric test in step (c) is carried out on a sample of plant tissue from the plantlets obtained.

13. Method according to Claim 2, **characterized in that** the gene of interest is a gene of interest which is expressed at a late stage of development.

14. Method according to any one of Claims 1 to 13, **characterized in that** the plant cells are plant cells obtained from a major crop species selected from rape, cauliflower, sunflower, wheat, maize, barley, tobacco, soya or from a vegetable species such as tomato, melon, lettuce or onion.

15. Method according to any one of Claims 1 to 14, **characterized in that** the plant cells are cells of cotyledons, hypocotyls or floral scapes.

16. Method according to any one of Claims 1 to 15, **characterized in that** the plant cells do not endogenously produce oxalate oxidase.

17. Method according to any one of Claims 2 to 16, **characterized in that** the protein of interest is an endochitinase.

18. Method according to any one of Claims 2 to 16, **characterized in that** the protein of interest is selected from the group consisting of a protein involved in the metabolic pathway of oils, starches, proteins, amino acids, lignin and components of the cell wall, or in the pathway for resistance to pathogens.

19. Method according to any one of Claims 2 to 16, **characterized in that** the protein of interest confers to plants resistance to pathogenic agents.

## Patentansprüche

1. Verfahren zur Herstellung von transgenen Pflanzen, die ein Protein exprimieren, das eine H₂O₂₋produzierende Aktivität besitzt, **dadurch gekennzeichnet, daß** es die folgenden Schritte umfaßt:
(a) Transformation von Pflanzenzellen oder Explantaten mit *Agrobacterium rhizogenes,* das einen Vektor, der ein für ein H₂O₂₋produzierendes Protein codierendes Gen trägt, in einem Zusammenhang, der seine Expression in dem Pflanzenmaterial gestattet, enthält, wobei diese Transformation die Bildung von transformierten Wurzeln induziert;
(b) Selektion von transformierten Wurzeln, die dieses Gen enthalten und exprimieren, mittels eines colorimetrischen Peroxidasetests;
(c) Regeneration von Pflänzchen aus selektierten Wurzeln und Überprüfung der Expression des Gens, das für ein H₂O₂-produzierendes Protein codiert, in den erhaltenen Pflänzchen, wobei diese Überprüfung der Expression mit einem colorimetrischen Peroxidasetest durchgeführt wird;
(d) phänotypisches Screening und gegebenenfalls molekulare Analyse der Nachkommenschaft der transgenen Pflanzen, was eine Selektion oder Bestätigung von erhaltenen transgenen Pflanzen, die nur das Transgen und nicht die T-DNA von *A. rhizogenes* enthalten, gestattet.

2. Verfahren zur Herstellung von transgenen Pflanzen, die ein interessierendes Gen und ein Gen, das für ein Protein mit H₂O₂-produzierender Aktivität codiert, exprimieren, **dadurch gekennzeichnet, daß** es die folgenden Schritte umfaßt:
(a) Transformation von Pflanzenzellen oder Explantaten mit *Agrobacterium rhizogenes,* das eine rekombinante DNA, die gleichzeitig ein für ein H₂O₂-produzierendes Protein codierendes Gen enthält und ein Gen, das für ein interessierendes Protein codiert, in einem Zusammenhang, der seine Expression in dem Pflanzenmaterial gestattet, enthält, wobei diese Transformation die Bildung von transformierten Wurzeln induziert;
(b) Selektion von transformierten Wurzeln, die das Gen, das für das interessierende Protein codiert enthalten und das Gen, das für ein H₂O₂-produzierendes Protein codiert exprimieren, mittels eines colorimetrischen Peroxidasetests;
(c) Regeneration von Pflänzchen aus selektierten Wurzeln und Überprüfung der Expression des Gens, das für ein H₂O₂-produzierendes Protein codiert, in den erhaltenen Pflänzchen, wobei diese Überprüfung der Expression mit einem colorimetrischen Peroxidasetest durchgeführt wird;
(d) phänotypisches Screening und gegebenenfalls molekulare Analyse der Nachkommenschaft der transgenen Pflanzen, um diejenigen Pflanzen, die nur das Transgen und nicht die T-DNA von *A. rhizogenes* enthalten, zu selektieren oder zu bestätigen;
(e) gegebenenfalls Aufreinigung des produzierten interessierenden Proteins.

3. Verfahren nach einem der Ansprüche 1 oder 2, **dadurch gekennzeichnet, daß** der colorimetrische Peroxidasetest gemäß Schritt (b) oder (c) in Gegenwart eines Substrats für das Protein mit H₂O₂₋produzierender Aktivität sowie in Gegenwart von Peroxidase zum Nachweis der H₂O₂-Bildung durchgeführt wird.

4. Verfahren nach einem der Ansprüche 1 bis 3, **dadurch gekennzeichnet, daß** es sich bei dem H₂O₂₋produzierenden Protein um ein Protein mit Oxalatoxidaseaktivität handelt.

5. Verfahren nach Anspruch 4, **dadurch gekennzeichnet, daß** das Protein mit. Oxalatoxidaseaktivität aus der Gruppe Oxalatoxidase aus Gerste, aus Sorghum, aus dem Moos *Mnium menziesii* oder Weizenkeim stammt.

6. Verfahren nach einem der Ansprüche 1 bis 5, **dadurch gekennzeichnet, daß** der colorimetrische Test in Schritt (b) an einer Wurzelprobe durchgeführt wird.

7. Verfahren nach einem der Ansprüche 1 bis 5, **dadurch gekennzeichnet, daß** der colorimetrische Test in Schritt (b) an einem flüssigen Inkubationsmedium nach Entfernung der Agrobakterien durchgeführt wird.

8. Verfahren nach einem der Ansprüche 1 bis 5, **dadurch gekennzeichnet, daß** der colorimetrische Test in Schritt (b) an dem von den transformierten Pflanzen auf einem mit Agar verfestigtem Medium oder auf einer Membran hinterlassenem Abdruck durchgeführt wird.

9. Verfahren nach einem der Ansprüche 1 bis 8, **dadurch gekennzeichnet, daß** die Selektion in Schritt (b) in Gegenwart von Oxalsäure als Substrat für das Protein mit H₂O₂-produzierender Aktivität durchgeführt wird.

10. Verfahren nach Anspruch 9, **dadurch gekennzeichnet, daß** die Oxalsäurekonzentration zwischen 5 und 50 mM beträgt.

11. Verfahren nach Anspruch 10, **dadurch gekennzeichnet, daß** die Oxalsäurekonzentration 15 mM beträgt.

12. Verfahren nach einem der Ansprüche 1 bis 5, **dadurch gekennzeichnet, daß** der colorimetrische Test von Schritt (c) an einer Probe von Pflanzengewebe der erhaltenen Pflänzchen durchgeführt wird.

13. Verfahren nach Anspruch 2, **dadurch gekennzeichnet, daß** es sich bei dem interessierenden Gen um ein interessierendes Gen, das während eines späten Entwicklungsstadiums exprimiert wird, handelt.

14. Verfahren nach einem der Ansprüche 1 bis 13, **dadurch gekennzeichnet, daß** es sich bei den pflanzlichen Zellen um pflanzliche Zellen handelt, die von einer Hauptkulturart aus der Reihe Raps, Blumenkohl, Sonnenblume, Weizen, Mais, Gerste, Tabak, Soja, oder um eine Gemüseart wie Tomate, Melone, Salat, Zwiebel handelt.

15. Verfahren nach einem der Ansprüche 1 bis 14, **dadurch gekennzeichnet, daß** es sich bei den pflanzlichen Zellen um Zellen von Kotyledonen, Hypokotylen oder Blütenschäften handelt.

16. Verfahren nach einem der Ansprüche 1 bis 15, **dadurch gekennzeichnet, daß** die pflanzlichen Zellen endogen keine Oxalatoxidase produzieren.

17. Verfahren nach einem der Ansprüche 2 bis 16, **dadurch gekennzeichnet, daß** es sich bei dem interessierenden Protein um eine Endochitinase handelt.

18. Verfahren nach einem der Ansprüche 2 bis 16, **dadurch gekennzeichnet, daß** das interessierende Protein aus der Gruppe Protein, das am Metabolismus von Ölen, Stärken, Proteinen, Aminosäuren, Lignin, Zellwandbausteinen oder an der Resistenz gegen Pathogene beteiligt ist, stammt.

19. Verfahren nach einem der Ansprüche 2 bis 16, **dadurch gekennzeichnet, daß** das interessierende Protein den Pflanzen eine Resistenz gegenüber pathogenen Agenzien vermittelt.
